# EUROPEAN PATENT SPECIFICATION

(11) **EP 1 765 272 B1**
(45) Date of publication and mention of the grant of the patent: **16.12.2009**
(21) Application number: 05766943.4
(22) Date of filing: 06.06.2005
(51) Int. Cl.: A61Q 19/00, A61K 8/44, A61K 8/84, A61Q 7/00

(54) **METHOD OF DECREASING SEBUM PRODUCTION AND PORE SIZE**
VERFAHREN ZUR VERMINDERUNG DER TALGPRODUKTION UND PORENGRÖSSE
PROCEDE DE DIMINUTION DE LA FABRICATION DE SEBUM ET DU DIAMETRE DES PORES

(30) Priority: 14.06.2004 US 579607 P
(43) Date of publication of application: 28.03.2007
(73) Proprietor: Unilever PLC, London EC4Y 0DY (GB); Unilever N.V., 3013 AL Rotterdam (NL)
(72) Inventor: BARTOLONE, John B., Unilever Home & Pers. Care USA, Trumbull, Connecticut 06611 (US); SHORE, Leonard,Unilever Home & Pers. Care USA, Trumbull, Connecticut 06611 (US); BAJOR, John S., Unilever Home & Pers. Care USA, Trumbull, Connecticut 06611 (US)
(74) Representative: Acham, Nicholas Clive
(86) International application number: PCT/EP2005/006119
(87) International publication number: WO 2005/120451

(56) References cited:
- WO-A-93/08790
- WO-A-99/03823
- WO-A-99/51213
- WO-A-02/053519
- WO-A-03/015729
- WO-A-03/063851
- WO-A-2005/013932
- US-A1- 2003 229 141

## Description

### FIELD OF THE INVENTION

The present invention relates to a method of decreasing sebum production in sebocytes and/or reducing pore size in skin. More specifically, the present invention relates to a method of decreasing sebum production and/or pore size by contacting skin with materials having hair growth suppression activity.

### BACKGROUND OF THE INVENTION

Sebum is skin oil which is produced by sebocytes (cells of the sebaceous glands in the skin) and is then secreted to the skin surface. Sebum is made up of a number of components, with about 57 % being triglycerides, or fatty adds. About 12 % of sebum is squalene, about 3 % of sebum are sterol esters, about 25 % wax esters, and about 1 to about 2 % cholesterol. A frequent and undesirable skin condition is "oily skin," the condition which results from the excessive amount of sebum on the skin. Oily skin is associated with a shiny, undesirable appearance and a disagreeable tactile sensation and affects various age groups. Excessive sebum production is cosmetically undesirable and has been associated with the skin condition acne. Therefore, cosmetic products and methods that provide sebum control are highly desirable.

Likewise, products and methods that reduce the size of skin pores are highly desirable. Pores are microscopic openings in skin that provide a way for oil or sebum to lubricate and protect the skin surface. Glands enlarge during puberty and there is a concomitant increase in the amount of oil produced. Consumers report that their pores get bigger to handle the increased output, although the true mechanisms controlling pores remain unknown at present. According to Van Scott, et al., as the growth of a hair is intimately dependent upon its papilla enveloped by the hair bulb, it was found that the size and mitotic activity of the matrix of the hair bulb normally can be correlated with the size and number of cells in the papilla. See "Geometric Relationships Between the Matrix of the Hair Bulb and Its Dermal Papilla in Normal and Alopecic Scalp," Presented at the Nineteenth Annual Meeting of The Society of Investigative Dermatology, Inc., June 21, 1958.

The present invention is based on the unexpected discovery that inhibition of hair growth inhibits, reduces, or controls sebum production and/or pore size.

Inhibition of hair growth is a subject discussed in numerous publications, including US 6 646 149 (Vermeulin et al), US 6 172 261 (Bums et al), US Published Patent Application 2003/0187276 (Oridigm Corporation), etc. Difluoryl methyl ornithine (DFMO), is a hair inhibitor that has been sold under the Veniqa™ brand from Bristol Myers Squibb (New Jersey). However, prior to the present invention, the use of certain agents known as hair growth inhibitors for reducing sebum production and pore size in skin has not been identified or suggested.

WO 03/063851 A1 discloses a composition for combating hair loss in man comprising spermidine.

US 2003/0229141 A1 discloses a method to alleviate or improve various cosmetic conditions and dermatological disorders using compositions comprising N-acetyl cysteine.

WO 03/015729 A1 discloses a topical composition for follicular delivery of an ornithine decarboxylase inhibitor.

WO 99/51213 A2 discloses the use of non-toxic polyamines in the palliative treatment of chronic diseases and disorders of epithelial tissue.

WO 93/08790 A1 discloses a treatment of acne or of psuedofolliculitis barbae by applying to the skin of the patient an inhibitor of ornithine decarboxylase.

### SUMMARY OF THE INVENTION

It has surprisingly been discovered that certain agents that inhibit hair growth are useful for inhibiting sebum production and/or reducing pore size. These discoveries have been utilized to provide the present invention, which includes cosmetic methods for decreasing sebum production in skin and/or reducing pore size.

In one aspect, the invention provides a method of decreasing sebum synthesis in a sebocyte or in skin of an individual in need thereof. The method comprises contacting the skin with a pore size reducing and/or sebum production inhibiting amount of hair growth inhibitor.

In another aspect, the invention provides a method of inhibiting sebum production and reducing pore size using certain agents, known to inhibit hair growth, in combination with other skin benefit actives by contacting the agents with skin. Agents useful according to the present invention include, but are not limited to: polyamine derivatives; DFMO; cosmetically acceptable salts thereof; solvates thereof; and mixtures thereof.

In particular, the present invention is directed to a cosmetic method of inhibiting sebum production and/or redudng pore size using a polyamine derivative and/or analog (PA analogs) that comprises a hydrophobic moiety covalently attached to a polyamine moiety. These PA analogs can be considered to have amphipathic character (hydrophobic as well as charged portions). The PA analogs and derivatives include those that may be viewed as a polyamine acylated with a hydrophobic acyl group, where acylation is by formation of either an amide or a sulfonamide linkage. The linkage between the hydrophobic acyl group and the polyamine moiety may occur at any amine group within the polyamine. Specifically, PA analogs according to the present invention are represented by general formula I:

R-X-polyamine (I)

and/or general formula II:

R-X-L-polyamine (II)

wherein R is selected from H or from the group of a straight or branched C₁₋₅₀ saturated or unsaturated aliphatic, carboxyalkyl, carbalkoxyalkyl, or alkoxy; a C₁₋₈ alicyclic; a single or multiring aryl substituted aliphatic; an aliphatic-substituted single or multiring aromatic; a single or multiring heterocyclic; a single or multiring heterocyclic aliphatic; a C ₁₋₁₀ alkyl; an aryl sulfonyl; or cyano;
"X" is -CO-, --SO₂--, or -CH₂- ;
"polyamine" is a naturally occurring or synthetically produced polyamine;
and, where applicable, L is a covalent bond or a naturally occurring amino add, omithine, 2,4- diaminobutyric add, or derivatives thereof.

The invention also provides, in another aspect, a composition for redudng sebum production and/or redudng pore size, comprising a hair growth inhibiting agent in combination with a sebum production inhibiting agent and a cosmetically acceptable carrier. The hair growth inhibitor comprises about 0.0001 % to about 50 % of the composition. The inventive compositions may additionally include another skin benefit agent.

In another aspect, the present invention is a cosmetic system for reducing sebum production and/or reducing pore size in skin, comprising said cosmetic composition, a container for said composition, and instructions for applying said composition to said skin.

Optional additional sebum production inhibitors include but are not limited to: carboxyalkylates of branched alcohols and ethoxylates thereof, triclosan, cerulenin, epigallocatechin gallate (EGCG), α-methylene-γ-butyrolactone, mixtures thereof, and analogs thereof, as well as cosmetically acceptable salts or solvates thereof.

### DETAILED DESCRIPTION OF THE INVENTION

The invention provides methods and cosmetic compositions for reducing sebum production and pore size using agents that inhibit hair growth. The present invention takes advantage of the discovery that the inhibition of hair growth using certain agents therefor results in a decrease in sebum production in sebocytes and/or follicle size and/or pore size reduction in skin.

Accordingly, in one aspect, the invention provides a method of decreasing sebum production in a sebocyte and/or reducing pore size via the inhibition of hair growth in the skin of an individual. The method comprises contacting the skin with a sebum production inhibiting and/or pore size reducing amount of hair growth inhibitor, thereby reducing sebum synthesis in the sebocyte and/or pore size in the skin.

As used herein, the term "comprising" means including, made up of, composed of, consisting and/or consisting essentially of. Furthermore, in the ordinary meaning of "comprising," the term is defined as not being exhaustive of the steps, components, ingredients, or features to which it refers.

Except in the operating and comparative examples, or where otherwise explicitly indicated, all numbers in this description indicating amounts or ratios of material or conditions of reaction, physical properties of materials and/or use are to be understood as modified by the word "about". The term "about" is used herein to mean approximately, in the region of, roughly, or around. When the term "about" is used in conjunction with a numerical range, it modifies that range by extending the boundaries above and below the numerical values set forth.

### Hair Growth Inhibitors As Agents For Sebum Suppression And/Or Pore Size Reduction

Hair inhibitors are selected for sebum suppression and/or pore size reduction from the group consisting of polyamine derivatives and/or analogs, including pharmaceutically acceptable salts and solvates thereof; DFMO; and mixtures thereof.

### Polyamine Derivatives and/or Analogs

In particular, the present invention is directed to a cosmetic method of inhibiting sebum production and/or redudng pore size using a hair growth inhibitor that is a polyamine derivative and/or analog (PA analogs) that comprises a hydrophobic moiety covalently attached to a polyamine moiety. These PA analogs can be considered to have amphipathic character (hydrophobic as well as charged portions). The PA analogs and derivatives include those that may be viewed as a polyamine acylated with a hydrophobic acyl group, where acylation is by formation of either an amide or a sulfonamide linkage. The linkage between the hydrophobic acyl group and the polyamine moiety may occur at any amine group within the polyamine.

As used herein, the term "polyamine" includes putrescine, spermine or spermidine, as well as longer linear polyamines, branched polyamines, and the like, which may have between 2 and about 10 nitrogens. Also included in this definition are polyamine derivatives or analogs comprising a basic polyamine chain with any of a number of functional groups bound to a C atom or a terminal or internal N atom. For modification at a primary amino group, a polyamine must, of course, contain such a group.

Polyamine "analogs" and/or "derivatives" generally refer to any modified polyamine molecule disclosed or described herein. These molecules are generally modifications of existing polyamines, whether naturally occurring or synthetically produced and may also be referred to as "polyamine agents", "PA" or "agents" of the invention. Preferred PAs bind and/or inhibit cellular polyamine transport, and as such may also be referred to as "transport binding molecules" or "polyamine transport inhibitors". The scope of this definition includes any modification to produce a PA from an existing polyamine or the isolation of a structurally identical PA from a naturally occurring source. Preferably, the modification is the addition of one or more chemical moieties to the polyamine.

The polyamine analogs and derivatives according to the present invention include those of the following general formula I:

R-X-polyamine (I)

wherein R is selected from H or from the group of a straight or branched C₁₋₅₀ saturated or unsaturated aliphatic, carboxyalkyl, carbalkoxyalkyl, or alkoxy; a C₁₋₈ alicyclic; a single or multiring aryl substituted aliphatic; an aliphatic-substituted single or multiring aromatic; a single or multiring heterocyclic; a single or multi-ring heterocyclic aliphatic; a C₁₋₁₀ alkyl; an aryl sulfonyl; or cyano;
"X" may be -CO-, -SO₂-, or -CH₂-, and
"polyamine" may be any naturally occurring, such as putresdne, spermine or spermidine, or synthetically produced polyamine.

Preferably, R is at least about C₅, at least about C₁₀, at least about C₁₁, at least about C₁₂, at least about C₁₃, at least about C₁₄, at least about C₁₅, at least about C₁₆, at least about C₁₇, at least about C₁₈, at least about C₁₉, at least about C₂₀, or at least about C₂₂.

The linkage between X and the polyamine may be direct, wherein there are no atoms between X and the nitrogen of the amine group of the polyamine, or indirect, where there may be one or more atoms between X and the nitrogen of the amine group of the polyamine. The linkage between X and the polyamine may occur via any amino group within the polyamine, although a primary amino group is used in preferred embodiments of the invention.

In preferred embodiments of the invention where the linkage between X and the polyamine is indirect, the intervening one or more atoms are preferably those of an amino acid or a derivative thereof. In particularly preferred embodiments of this type, the intervening one or more atoms are those of lysine, aspartic acid, glutamic acid, omithine, or 2,4-diaminobutyric acid. Preferred compounds of this type may be represented by the following general formula II:

R-X-L-polyamine (II)

wherein R is a straight or branched C₁₀₋₅₀ saturated or unsaturated aliphatic, carboxyalkyl, carbalkoxyalkyl, or alkoxy; a C₁₋₈ alicyclic; a single or multiring aryl substituted or unsubstituted aliphatic; an aliphatic-substituted or unsubstituted single or multiring aromatic; a single or multiring heterocyclic; a single or multiring heterocyclic aliphatic; an aryl sulfonyl;
X is -CO-, -SO₂-, or -CH₂-; and L is a covalent bond or a naturally occurring amino add, ornithine, 2,4-diaminobutyric add, or derivatives thereof.

The analogs and derivatives of the invention, may be optionally further substituted at one or more other positions of the polyamine. These include, but are not limited to, internal nitrogen and/or internal carbon atoms. In one aspect of the invention, preferred substituents are structures that increase polyamine transport inhibition, binding affinity or otherwise enhance the irreversibility of binding of the compound to a polyamine binding molecule, such as the polyamine transporter, an enzyme or DNA. Such additional substituents include the aziridine group and various other aliphatic, aromatic, mixed aliphatic- aromatic, or heterocyclic multi-ring structures. Reactive moieties which, like aziridine, bind covalently to a polyamine transporter or another polyamine binding molecule, are also within the scope of this invention. Examples of reactive groups that react with nucleophiles to form covalent bonds include chloro-, bromo- and iodoacetamides, sulfonylfluorides, esters, nitrogen mustards, etc. Such reactive moieties are used for affinity labeling in a diagnostic or research context, and may contribute to pharmacological activity in inhibiting polyamine transport or polyamine synthesis. The reactive group can be a reactive photoaffinity group such as an azido or benzophenone group. Chemical agents for photoaffinity labeling are well-known in the art (US 2003/0187276).

### Inhibitors of Ornithine Decarboxylase (ODC)

Ornithine decarboxylase (ODC) is an enzyme that plays a role in synthesizing polyamines, by decarboxylating omithine, which synthesizes putredne. This is the rate-limiting step in produdng polyamines (i.e., putrecine to spermidine to spermine). Inhibition of ODC inhibits polyamine production or biosynthesis, thereby inhibiting cell growth and proliferation. One aspect of the present invention is based on the discovery that inhibition of ODC reduces the size of sebaceous glands and leads to reduction of sebum synthesis and/or to reduction of appearance of pore size in skin.

Inhibitors of ODC include difluoryl methyl ornithine (DFMO), available under the Vaniqa™ brand, methylglyoxalbisguanylhydrazone (MGBG), hydrozino ornithine (HAVA), and mixtures thereof. Preferred among these is DFMO due to its commercial availability.

The phrase "inhibiting" as used herein refers to about 10% to about 100% decrease in the activity to which it refers. More preferably, the term "inhibiting" refers to about a 25% to about a 100% decrease in activity, and most preferably, to about a 50% to about a 100% decrease in activity to which it refers. A decrease or change in activity can be measured by any method known to or developed by one skilled in the art.

The term "educing the appearance of oily or greasy skin and/or pore size" is meant herein to refer to any detectable reduction in skin sebum and/or pore size, e.g., a reduction visible to the naked eye, that occurs after contacting the skin of an individual with a treatment regimen comprising an inhibitor of hair growth and/or sebum production.

The term "reducing sebum production" is used herein to mean a detectable lowering of the amount of sebum synthesized by a sebocyte exposed to a compound that inhibits hair growth and/or sebum production as compared to the amount of sebum synthesized in the absence of such an inhibiting compound. The term "reduction" as used herein in relation to sebum and/or sweat and/or pore size means the complete prevention, control of secretion, or a degree of reduction of the formation of sebum and/or sweat and/or pore size, respectively. The term "lowering" preferably refers to 5 about a 10% to about a 100% decrease in the amount of sebum observed or measured. More preferably, the term "lowering" refers to about a 25% to about a 100% decrease in the amount of sebum and/or size of pores observed or measured. Most preferably, the term "lowering" refers to about a 50% to about a 100% decrease in the amount of sebum and/or size of pores observed or measured. The terms lowering, reducing, decreasing, suppressing and inhibiting when used in relation to sebum production are intended to be used interchangeably.

As used herein, the term "a person in need thereof" refers to an individual with a normal but noticeable and undesired oily and/or porous skin condition or an individual that elects to decrease amount of sebum and/or size of pores in the absence of a noticeable and undesired oily and/or porous skin condition.

As used herein, skin pores are defined as openings or troughs on the skin surface. More particularly, a pore is an opening for a sebaceous oil gland. Pores are microscopic openings in skin that provide a way for oil or sebum to lubricate and protect the skin surface. Glands enlarge during puberty and there is a concomitant increase in the amount of oil produced. The overall appearance of pores depends on the depth and diameter of the troughs as well as on the surrounding skin color, texture and periodicity of the pores.

As used herein, a "sebum and/or pore size reducing effective amount" of a compound means an amount of the compound that detectably reducing sebum production and/or pore size in skin after a cosmetically effective period of time. One skilled in the art is able to determine a "cosmetically effective period time" based on the particular skin oil and/or pore size reducing effect desired.

The term "skin" as used herein includes the skin on or in the face, mouth, neck, chest, back, arms, hands, legs, and scalp.

Preferably, the methods and compositions of the invention are for application to a vertebrate cell or individual, more particularly to a mammalian cell or individual, and most preferably to a human cell or individual. The term "individual" is used is herein to refer to a vertebrate, a mammal or a human.

### Optional Sebum Production and/or Pore Size Reducing Agents

In some embodiments, additionally but optionally, a further sebum production inhibitor and/or pore size reducing agent is selected from the group consisting of carboxyalkylates of branched alcohols and/or alkoxylates thereof (e.g., tridecyl carboxy alkylates), cerulenin and a cerulenin analog, including pharmaceutically acceptable salts and solvates thereof. In other embodiments, the fatty add synthase (FAS) inhibitor is triclosan or analogs thereof. As used herein, the term "analog" refers to a chemical compound that is structurally related to cerulenin and retains at least a measurable amount of sebum production inhibitory activity. Triclosan is known to inhibit enoyl-reductase of type I fatty acid synthase. In a further embodiment, the sebum production inhibitor and/or pore size reducer is EGCG, a polyphenolic compound that is a green tea extract available from Sigma Corporation (St. Louis, Missouri), or α-methylene-γ-butyrolactone.

By way of a non-limiting example, cerulenin is a sebum production inhibitor useful in the methods of the invention. Structurally, cerulenin is characterized as [2R,3S]-2,3-epoxy-4-oxo-7, 10-trans,trans-dodecanoic add amide. Cerulenin was originally isolated as a potential antifungal antibiotic from the culture broth of *Cephalosporium caerulens*. The sebum production inhibitor is selected from the group consisting of cerulenin and a cerulenin analog, including cosmetically acceptable salts and solvates thereof. Non-limiting examples of cerulenin and cerulenein analogs include those described in US 5 539 132 (Royer et al). Alternatively cerulenin may be obtained commercially from Sigma Corporation (St. Louis, Missouri).

### Carboxyalkylates of Branched Alcohols and/or Alkoxylates Thereof

Carboxyalkylates of branched alcohols and/or alkoxylates thereof include compounds of formula A and compositions including the compounds:

R-O-M (A)

wherein:
R is a branched alkyl or alkenyl chain having at least 7 carbon atoms, and at least two branches;
O is an oxygen atom; and
M is (-(CH₂)ₚO)ₙ-(CH₂)ₘCO₂X)
where n is 0 or an integer between 1 and 7, m is an integer between 1 and 4, p is an integer between 2 and 4; and X is hydrogen, a methyl group, an ethyl group, or a cation. The cation is selected from the group consisting of sodium, lithium, potassium, calcium, copper, magnesium, manganese, strontium, sulfur, zinc, and amines. Preferably, X is hydrogen or a cation.

Preferred compounds of this type are tridecyl carboxy alkylates, more preferably, tridecyl carboxy methylates and/or tridecyl carboxy ethylates.

### Identifying Sebum Production Inhibitors

To determine if a hair growth inhibitor is useful in the methods of the invention, any assay known to those with skill in the art which can demonstrate a reduction in sebum production and/or pore size may be used. For example, cultured sebocytes can be incubated with a proposed inhibitor test compound and tested for sebum content, e.g., as described with reference to the assay herein below. This sebum content is then compared with the sebum content of untreated, cultured sebocytes to determine if the hair growth inhibitor inhibits sebum production.

In a non-limiting example, sebum production may be assayed in human primary sebocytes according to the following protocol. The results will show the percent reduction in total lipids produced by sebocytes in culture with sebum control agents relative to a control sample which has only been incubated with vehicle.

### In Vitro Sebocyte Lipogenesis Assay

Human sebaceous glands were isolated from the nose of a male (age 60) and cultured using submerged tissue culture techniques (Bajor et al, J. Invest. Dermatol., 102:1994, p.564). These sebocytes accumulate intracellular lipid droplets characteristic of mature human sebum.

Sebocytes are added to each well of a 96 well tissue culture plate and incubated at 37°C in the presence of 7.5% CO₂ for 10 days. The growth medium is changed three times per week. On the day of experimentation, the growth medium is removed and the sebocytes washed three times with phosphate buffered saline (PBS). Fresh phenol-free sebocyte growth media in 0.2 ml amounts containing various concentrations of active agent speculated to inhibit lipogenesis is added to each well. Triplicate wells are utilized for each sample. Controls consist of PBS, dimethyl sulfoxide (DMSO) or ethanol used to solubilize the lipophilic compounds, and phenol red, a compound which possesses estrogen-like activity. The cultures are incubated at 37°C in the presence of 7. 5% by volume CO₂ for 24 hours. A radioactive label is prepared by adding 100 µl (micro-liters) of 14-C labelled acetic acid (Amersham, sodium salt, specific activity of 56 mCi/mmol) to 10 ml of 50 mM sodium acetate buffer. Then 50 µl is added to each well containing the sebocytes and active agents. The cultures are returned to the incubator for 4 hours. Hereafter the treatments and label are removed and the sebocytes rinsed three times with fresh PBS. Cells containing the 14-C label are extracted and the label counted using a Beckman scintillation counter.

For each 96 well tissue culture plate, 20 samples could be analyzed. Of these, 1 sample is reserved for media, 1 sample for DMSO or ethanol, and 1 sample for phenol red leaving 17 remaining samples.

### Cosmetic Methods And Compositions for Reducing Appearance Of Skin Oiliness and/or Pore Size

Contact of sebocytes, either *in vitro* or *in vivo*, with an amount of a certain inhibitor of hair growth that is effective to inhibit sebum production, will result in the desired sebum and/or pore reducing effect. Suitable compounds for this purpose include those identified above. In addition, optional sebum reducing agents include, but are not limited to, those described above. Cosmetic compositions of the invention include the sebum and pore reducing agents and may be administered to a human or animal having a condition that normally occurs in skin, of a type that causes over-production of sebum.

The compositions and methods of the current invention are noticeable for cosmetic purposes. For example, occurrences in the skin or hair of noticeable but undesired feel or appearance of oiliness as a result of sebum production or overproduction may be ameliorated using the methods of the present invention. Cosmetic applications for methods of the present invention include the application of compositions containing one or more compounds that decreases sebum production in a sebocyte to enhance or otherwise alter the visual appearance of skin or hair. Alternatively, the prevention of sebum production, for example as a result of sweating or perspiration, is also contemplated as an appropriate application of the cosmetic methods of the invention for reducing the appearance of oily skin and/or pore size.

Compounds for reducing the appearance of oily skin and/or pore size are used in the inventive compositions in amounts of 0.00001 to 50 %, preferably 0.1 to 20 %, more preferably 1 to 15 %, most preferably 2 to 5 % by weight of the composition.

### Optional Additional Skin Benefit Agents

Various types of additional active ingredients may be present in the cosmetic compositions of the present invention. Actives are defined as skin benefit agents other than emollients and other than ingredients that merely improve the physical characteristics of the composition. Although not limited to this category, general examples include additional oily skin appearance enhancing ingredients such as talcs and silicas, as well as alpha-hydroxy acids, beta-hydroxy acids, poly-hydroxy acids, benzoyl peroxide, astringent salts such as zinc salts, retinoids, sunscreens, and preservatives.

Beta-hydroxy acids include salicylic acid, for example. Zinc pyrithione is an example of zinc salts useful in the compositions of the present invention.

Optionally, but preferably, astringent salts are included in the compositions of the present invention. The astringent salts may be inorganic or organic salts of aluminum, zirconium, zinc and mixtures thereof. Preferably, the astringent salts are employed herein in particulate form, i.e., hydrophilic porous particles, of less than about 100 microns in size, preferably about 3 microns to about 10 microns in size. Salts useful as astringents or as components of astringent aluminum complexes include aluminum hydroxide, aluminum halides, aluminum hydroxyhalides, zircon oxyhalides, zirconyl hydroxyhalides and mixtures of these salt materials.

Aluminum salts of this type include aluminum chloride and the aluminum hydroxyhalides having the general formula Al₂(OH)ₓQ_{y}-XH₂O where Q is chlorine, bromine or iodine, where x is 2 to 5 and x+y = 6 and x and y do not need to be integers; and where X is about 1 to 6. For example, aluminum chlorohydrate, having the formula [Al₂(OH)₅Cl]-XH₂O, is preferred, due to its ready commercial availability and relatively low cost.

Several types of complexes utilizing the above astringent salts are known in the antiperspirant art. For example US 3 792 068 (Luedders et al) discloses complexes of aluminum, zirconium and amino acids such as glycine (ZAG complexes). The ZAG complexes ordinarily have an Al:Zr ratio of from 1.67 to 12.5 and a Metal:Cl ratio of from 0.73 to 1.93. The preferred amino acid for preparing such ZAG complexes is glycine of the formula CH₂(NH₂)COOH. Spherical ZAG, with particle size 1 to 100 microns, is especially preferred.

More specifically, the following is a list of astringent salts which may be useful for the present invention and which have approved listings under the United States Food & Drug Administration, Federal Register. They include aluminum chloride, aluminum chlorohydrate, aluminum chlorohydrex, aluminum chlorohydrex PEG, aluminum chlorohydrex PG, aluminum dichlorohydrate, aluminum dichlorohydrex PEG, aluminum dichlorohydrex PG, aluminum sesquichlorohydrate, aluminum sesquichlorohydrex PEG, aluminum sesquichlorohydrex PG, aluminum sulfate, aluminum zirconium octachlorohydrate, aluminum zirconium octachlorohydrex GLY (abbreviation for glycine), aluminum zirconium pentachlorohydrate, aluminum zirconium pentachlorohydrex GLY, aluminum zirconium tetrachlorohydrate, aluminum zirconium trichlorohydrate, aluminum zirconium tetrachlorohydrate GLY, and aluminum zirconium trichlorohydrate GLY.

Also suitable are: potassium aluminium sulphate, (also known as alum (KAl(SO₄)₂12H₂O)), aluminium undecylenoyl collagen amino add, sodium aluminium lactate+ aluminium sulphate (Na₂HAl(OOCCHOHCH₃)₂-(OH)₆) + Al₂(SO₄)₃), sodium aluminium chlorohydroxylactate, aluminium bromohydrate (Al₂Br(OH)₅nH₂O), aluminium chloride (AlCl₃6H₂O), complexes of zinc salt and of sodium salt, complexes of lanthanum and cerium, and the aluminium salt of lipoamino acids (R-CO-NH-CHR'-CO-OAl-(OH)₂with R = C₆₋₁₁ and R'=amino add).

Preferably, the antiperspirant is an aluminium salt and, more preferably, it is chosen from potassium aluminium sulphate (alum) and aluminium chlorohydrate.

Amounts of the active astringent salt may range from 0.000001 % to 20%, preferably from 0.10 % to 18 %, more preferably 1 to 15 %, and optimally 2 % to 3 % by weight of the composition.

Aluminum chlorohydrate (ACH, is the most preferred astringent salt for the purposes of the present invention, due to its wide commercial availability and relatively low cost.

Optionally, but preferably, the inventive compositions may also include a retinoid. Retinoids increase collagen synthesis by dermal fibroblasts. This results in smoothening of wrinkled skin. Addition of retinoids also provides improved inhibition of lipogenesis. The term "retinoids" as used herein includes retinoic acid, retinol, retinal, and retinyl esters. Included in the term "retinoic acid" are 13-cis retinoic acid and all-trans retinoic acid.

The term "retinol" as used herein includes the following isomers of retinol: all-trans-retinol, 13-cis-retinol, 11-cis-retinol, 9-cis-retinol, and/or 3,4-didehydro-retinol. The preferred isomer is all-trans-retinol, due to its wide commercial activity.

Retinyl ester is an ester of retinol. The term "retinol' has been defined above. Retinyl esters suitable for use in the present invention are C₁-C₃₀ esters of retinol, preferably C₂-C₂₀ esters, and most preferably C₂, C₃, and C₁₆ esters because they are more commonly available. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecanoate, retinyl laurate, retinyl tridecanoate, retinyl mytistate, retinyl pentadecanoate, retinyl heptadecanoate, retinyl stearate, retinyl isostearate, retinyl nonadecanoate, retinyl arachidonate, retinyl behenate, retinyl linoleate, retinyl oleate, retinyl lactate, retinyl glycolate, retinyl hydroxy caprylate, retinyl hydroxy laurate, retinyl tartarate.

The retinoids in the present invention are present in an amount of from 0.001% to 10%, preferably from 0.01% to 1%, and most preferably from 0.01% to 0.05% by weight of the composition.

Sunscreens include those materials commonly employed to block ultraviolet light. Illustrative compounds are the derivatives of para-aminobenzoic acid (PABA), cinnamate and salicylate. For example, avobenzophenone (Parsol 1789®), octyl methoxycinnamate (Parsol™ MCX) and 2-hydroxy-4-methoxy benzophenone (also known as oxybenzone and available as Benzophenone™ 3) can be used. The exact amount of sunscreen employed in the compositions can vary depending upon the degree of protection desired from the sun's UV radiation.

Many cosmetic compositions, especially those containing water, must be protected against the growth of potentially harmful microorganisms. Suitable preservatives include alkyl esters of p-hydroxybenzoic acid, hydantoin derivatives, propionate salts, and a variety of quaternary ammonium compounds. Particularly preferred preservatives of this invention are methyl paraben, propyl paraben, phenoxyethanol and benzyl alcohol. Preservatives will usually be employed in amounts ranging from 0.1 % to 2% by weight of the composition.

### Cosmetically Acceptable Vehicle

The compositions according to the invention comprise a cosmetically acceptable vehicle to act as a diluant, dispersant or carrier of the inventive skin benefit compounds and any optional skin benefit agents, so as to facilitate their distribution when the composition is applied to the skin.

The vehicle may be aqueous, anhydrous or an emulsion. Preferably, the compositions are aqueous or an emulsion, especially water-in-oil or oil-in-water emulsions. Water when present will be in amounts which may range from 5 to 99%, preferably from 40 to 90%, optimally between 60 and 90% by weight of the composition.

Besides water, relatively volatile solvents may also serve as carriers within compositions of the present invention. Most preferred are monohydric C₁-C₃ alkanols. These include ethyl alcohol, methyl alcohol and isopropyl alcohol. The amount of monohydric alkanol may range from 1 to 70%, preferably from 10 to 50%, optimally between 15 and 40% by weight of the composition.

Emollient materials may also serve as cosmetically acceptable carriers. These may be in the form of silicone oils and synthetic esters. Amounts of the emollients may range anywhere from 0.1 to 50%, preferably between 1 and 20% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms. Linear volatile silicone materials generally have viscosities less than about 5 centistokes at 25°C while cyclic materials typically have viscosities of less than about 10 centistokes. Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 25 million centistokes at 25°C. Among the preferred non-volatile emollients useful in the present compositions are the polydimethyl siloxanes having viscosities from about 10 to about 400 centistokes at 25°C.

Among the ester emollients are:
(1) Alkenyl or alkyl esters of fatty adds having 10 to 20 carbon atoms. Examples thereof include isoarachidyl neopentanoate, isononyl isonanonoate, oleyl myristate, oleyl stearate, and oleyl oleate.
(2) Ether-esters such as fatty add esters of ethoxylated fatty alcohols.
(3) Polyhydric alcohol esters such as ethylene glycol mono and di-fatty add esters, diethylene glycol mono- and di-fatty add esters, polyethylene glycol (200-6000) mono- and di-fatty add esters, propylene glycol mono- and di-fatty add esters, polypropylene glycol 2000 monooleate, polypropylene glycol 2000 monostearate, ethoxylated propylene glycol monostearate, glyceryl mono- and di-fatty add esters, polyglycerol poly-fatty esters, ethoxylated glyceryl monostearate, 1,3-butylene glycol monostearate, 1,3-butylene glycol distearate, polyoxyethylene polyol fatty add ester, sorbitan fatty add esters and polyoxyethylene sorbitan fatty add esters.
(4) Wax esters such as beeswax, spermaceti, myristyl myristate, stearyl stearate and arachidyl behenate.
(5) Sterols esters, of which cholesterol fatty add esters are examples.

Fatty acids having from 10 to 30 carbon atoms may also be included as cosmetically acceptable carriers for compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol type may also be employed as cosmetically acceptable carriers in compositions of this invention. The humectant aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably propylene glycol or sodium hyaluronate. The amount of humectant may range anywhere from 0.5 to 30%, preferably between 1 and 15% by weight of the composition.

Thickeners may also be utilized as part of the cosmetically acceptable carrier of compositions according to the present invention. Typical thickeners include crosslinked acrylates (e.g. Carbopol™ 982), hydrophobically-modified acrylates (e.g. Carbopol™ 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0001 to 5%, usually from 0.001 to 1 %, optimally from 0.01 to 0.5% by weight of the composition.

Collectively, the water, solvents, silicones, esters, fatty acids, humectants and/or thickeners will constitute the cosmetically acceptable carrier in amounts from 1 to 99.9%, preferably from 80 to 99% by weight of the composition.

An oil or oily material may be present, together with an emulsifier to provide either a water-in-oil emulsion or an oil-in-water emulsion, depending largely on the average hydrophilic-lipophilic balance (HLB) of the emulsifier employed.

### Use of the Novel Compositions

The compositions according to the invention are intended primarily as a product for topical application to human skin, especially as an agent for controlling or preventing excessive sebum secretion and/or for reducing pore size. Suppression of sebum provides multiple benefits, including: improved skin condition; reduction of an unpleasant appearance and feel of greasy skin; reduction and/or prevention of acne, rosacea, seborrhea, oily scalp, oily/greasy hair, and dandruff.

In use, a quantity of the composition, for example from 1 to 100 ml, is applied to exposed areas of the skin, from a suitable container or applicator and, if necessary, it is then spread over and/or rubbed into the skin using the hand or fingers or a suitable device.

Optionally, but preferably, the method includes a step of shaving the skin. More preferably, the skin is shaved prior to application of the inventive compositions.

The present invention also includes a cosmetic method of controlling or preventing an oily skin condition, especially in the facial area, by applying to the skin the inventive composition. In another aspect, the present invention includes a cosmetic method of controlling, preventing, or treating oily or greasy hair.

The invention also includes a cosmetic method of reducing, preventing or controlling sebum secretion from sebocytes by applying the inventive composition.

The invention also includes a cosmetic method of reducing or controlling the perception of oily or greasy skin by applying to the skin the inventive composition.

### Product Form and Packaging

The cosmetic skin composition of the invention can be in any form, e.g. formulated as a toner, gel, lotion, a fluid cream, or a cream. The composition can be packaged in a suitable container to suit its viscosity and intended use by the consumer. For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar. The invention accordingly also provides a closed container containing a cosmetically acceptable composition as herein defined. The composition may also be included in capsules such as those described in US 5 063 057.

The composition with the packaging, and together with a set of instructions associated with the package, may comprise a system for controlling/reducing skin sebum and/or reducing pore size. The set of instructions may be printed on the package or placed in the package. The set of instructions typically communicates to the consumer of the present articles to dispense the composition in an amount effective to provide a solution to problems involving, and/or provision of a benefit relating to, those selected from the group consisting of controlling/reducing sebum production, improving appearance, reducing pore size and/or a combination thereof. It is important that the consumer of the present article be aware of these benefits, since otherwise the consumer would not know that the composition would solve these problems or combination of problems and/or provide these benefits or combination of benefits.

### EXAMPLE 1

### Sebocyte Assay Procedure

Secondary cultures of human sebocytes obtained from an adult male were grown in 96-well tissue culture plates (Packard) until three days post-confluence. The sebocyte growth medium consisted of Clonetics Keratinocyte Basal Medium (KBM) supplemented with 14 *u*g/ml bovine pituitary extract, 0.4 *u*g/ml hydrocortisone, 5 *u*g/ml insulin, 10 ng/ml epidermal growth factor, 1.2 x 10⁻¹⁰ M cholera toxin, 100 units/ml penicillin, and 100 *u*g/ml streptomycin. All cultures were incubated at 37°C in the presence of 7.5% by volume CO₂. The medium was changed three times per week.

On the day of experimentation, the growth medium was removed and the sebocytes washed three times with sterile Dulbecco's Modified Eagle Medium (DMEM; phenol red free). Fresh DMEM was added to each sample (5 replicates) with 5-microliters of test agent (DL-α-difluoromethylomithine hydrochloride) solubilized in water. Controls consisted of addition of water alone. Each plate was returned to the incubator for 20-hours followed by the addition of ¹⁴C-acetate buffer (5 mM final concentration, 56 mCi/mmol spedfic activity). The sebocytes were returned to the incubator for 4 hours after which each culture was rinsed 3 times with phosphate buffered saline to remove unbound label. Radioactive label remaining in the sebocytes was determined using a Packard TopCount scintillation counter. Statistical significance (p value) was calculated using Student's t-test. The results that were obtained are summarized in table 1. Phenol Red, a known sebum suppressive agent, was employed as a positive control.

**TABLE 1**

| Treatment | % of Control | p-value |
|---|---|---|
| 10 uM DFMO | 85.2 | 0.221 |
| 10 uM DFMO | 95.5 | 0.111 |
| 1000 uM DFMO | 88.0 | 0.138 |
| 282 uM Phenol Red | 34.5 | 2.7 x 10⁻⁴ |
| 28.2 uM Phenol Red | 22.1 | 3.4 x 10⁻⁷ |

| | | |
|---|---|---|
| uM is an abbreviation for "micromolar' | | |

These results indicate limited sebum suppressive activity after 24-hours. The experiment was repeated as above with modifications. On the day of experimentation, the growth medium was removed and the sebocytes washed three times with sterile Dulbecco's Modified Eagle Medium (DMEM; phenol red free). Fresh DMEM was added to each sample (3 replicates) with 5-microliters of test agent (DL-a-difluoromethylomithine hydrochloride) solubilized in water. Controls consisted of addition of water alone. Each plate was returned to the incubator for 68-hours followed by the addition of ¹⁴C-acetate buffer (5 mM final concentration, 56 mCi/mmol specific activity). The sebocytes were returned to the incubator for 4-hours, followed by rinsing each culture 3 times with phosphate buffered saline to remove unbound label. Radioactive label remaining in the sebocytes was determined using a Packard TopCount scintillation counter. Statistical significance (p value) was calculated using Student's t-test. The results that were obtained are summarized in table 2 below.

**TABLE 2**

| Treatment | % of Control | p-value |
|---|---|---|
| 10 uM DFMO | 59.5 | 0.002 |
| 10 uM DFMO | 58.3 | 0.093 |
| 1000 uM DFMO | 41.4 | 0.071 |

These results indicate enhanced sebum suppressive activity after prolonged incubation with DFMO.

## Claims

1. A cosmetic method of decreasing sebum production in sebocytes and/or pore size in skin of an individual in need thereof, comprising contacting said sebocyte and/or skin with a composition comprising a sebum production and/or pore size inhibiting amount of an agent selected from the group consisting of polyamine derivatives; difluoryl methyl ornithine (DFMO); cosmetically acceptable salts thereof; solvates thereof; and mixtures thereof,
wherein said polyamine derivative is a compound of general formula I:
R-X-polyamine (I)
wherein R is selected from H or from the group of a straight or branched C₁₋₅₀ saturated or unsaturated aliphatic, carboxyalkyl, hydroxycarboxyalkyl, or alkoxy; a C₃₋₈ alicyclic; a single or multiring aryl substituted aliphatic; an aliphatic-substituted single or multiring aromatic; a single or multiring heterocyclic; a single or multiring heterocyclic substituted aliphatic; a C₁₋₁₀ alkyl; an aryl sulfonyl; or cyano;
"X" is -CO-, -SO₂-, or -CH₂-; and
"polyamine" is a naturally occurring or synthetically produced polyamine, or
wherein said agent is a polyamine derivative of general formula II:
R-X-L-polyamine (II)
wherein R is a straight or branched C₁₀₋₅₀ saturated or unsaturated aliphatic, carboxyalkyl, hydrocarboxyalkyl, or alkoxy; a C₃₋₈ alicyclic; a single or multiring aryl substituted or unsubstituted aliphatic; an aliphatic-substituted or unsubstituted single or multiring aromatic; a single or multiring heterocyclic; a single or multiring heterocyclic substituted aliphatic; an aryl sulfonyl;
X is -CO-, -SO₂-, or -CH₂-;
L is a covalent bond or a naturally occurring amino acid, ornithine, 2,4-diaminobutyric acid, or derivatives thereof; and
"polyamine" is a naturally occurring or synthetically produced polyamine.

2. The cosmetic method according to claim 1, further comprising shaving said skin.

3. The cosmetic method according to any one of the preceding claims, wherein said agent is DFMO.

4. The cosmetic method according to anyone of the preceding claims, wherein the composition further comprises a sebum production inhibitor selected from the group consisting of carboxyalkylates of branched alcohols and/or ethoxylates thereof, triclosan, cerulenin, epigallocatechin gallate (EGCG), α-methylene-γ-butyrolactone, mixtures thereof, and analogs thereof.

5. The cosmetic method according to anyone of the preceding claims, wherein said method reduces synthesis of squalene, triglycerides, free fatty acids, wax esters, cholesterol, sterol esters, and/or a combination thereof.

6. A cosmetic composition for reducing sebum production and/or pore size comprising:
a sebum production and/or pore size inhibiting effective amount of one or more agents selected from the group consisting of polyamine derivatives; DFMO; a cosmetically acceptable salt thereof; a solvate thereof; and mixtures thereof; and
a cosmetically acceptable carrier, and
a sebum production inhibitor selected from the group consisting of carboxyalkylates of branched alcohols and/or ethoxylates thereof, triclosan, cerulinin, epigallocatechin gallate (EGCG), α-methylene-γ-butyrolactone, mixtures thereof,
wherein said agent is a polyamine derivative of general formula I:
R-X-polyamine (I)
wherein R is selected from H or from the group of a straight or branched C₁₋₅₀ saturated or unsaturated aliphatic, carboxyalkyl, hydrocarboxyalkyl, or alkoxy; a C₃₋₈ alicyclic; a single or multiring aryl substituted aliphatic; an aliphatic-substituted single or multiring aromatic; a single or multiring heterocyclic; a single or multiring heterocyclic substituted aliphatic; a C₁₋₁₀ alkyl; an aryl sulfonyl; or cyano;
"X" is -CO-, -SO₂-, or -CH₂- ; and
"polyamine" is a naturally occurring or synthetically produced polyamine, or
wherein said agent is a polyamine derivative of general formula II:
R-X-L-polyamine (II)
wherein R is a straight or branched C₁₀₋₅₀ saturated or unsaturated aliphatic, carboxyalkyl, hydrocarboxyalkyl, or alkoxy; a C₃₋₈ alicyclic, a single or multiring aryl substituted or unsubstituted aliphatic; an aliphatic-substituted or unsubstituted single or multiring aromatic; a single or multiring heterocyclic; a single or multi-ring heterocyclic substituted aliphatic; an aryl sulfonyl;
X is -CO-, -SO₂-, or -CH₂-;
L is a covalent bond or a naturally occurring amino acid, ornithine, 2,4-diaminobutyric acid, or derivatives thereof; and
"polyamine" is a naturally occurring or synthetically produced polyamine.

7. The cosmetic composition according to claim 6, wherein said agent is difluorylmethylornithine (DFMO).

8. The cosmetic composition according to claim 6 or claim 7 wherein said hair growth inhibitor comprises 0.0001 % to 50% by weight of said composition.

9. The cosmetic composition according any one of claims 6 to 8, further comprising a skin benefit agent

10. A cosmetic system for reducing sebum production in skin, comprising said cosmetic composition according to any one of claims 6 to 9, a container for said composition, and instructions for applying said composition to said skin.

## Patentansprüche

1. Kosmetisches Verfahren zur Verminderung der Talgproduktion in Sebocyten und/oder der Porengröße in der Haut eines Individuums, das deren bedarf, umfassend In-Kontakt-Bringen des Sebocyten und/oder der Haut mit einer Zusammensetzung, die eine die Talgproduktion und/oder Porengröße inhibierende Menge eines Mittels umfasst, das ausgewählt ist aus der Gruppe, bestehend aus Polyamin-Derivaten; Difluorylmethylornithin (DFMO); kosmetisch verträglichen Salzen davon, Solvaten und Gemischen davon,
wobei das Polyamin-Derivat eine Verbindung der allgemeinen Formel I ist:
R-X-Polyamin (I)
worin R ausgewählt ist aus H und der Gruppe aus einer geradkettigen oder verzweigten, gesättigten oder ungesättigten aliphatischen C₁₋₅₀-Gruppe, -Carboxyalkyl-, -Hydroxycarboxyalkyl- oder -Alkoxy-Gruppe; einer alicyclischen C₃₋₈-Gruppe; einer monocyclischen oder polycyclischen Aryl-substituierten aliphatischen Gruppe; einer aliphatisch substituierten monocyclischen oder polycyclischen aromatischen Gruppe; einer monocyclischen oder polycyclischen heterocyclischen Gruppe; einer monocyclischen oder polycyclischen heterocyclischen substituierten aliphatischen Gruppe; einem C₁₋₁₀-Alkyl; einem Arylsulfonyl und Cyano;
"X" -CO-, -SO₂- oder -CH₂- ist, und
"Polyamin" ein natürlich vorkommendes oder synthetisch produziertes Polyamin ist oder
wobei das Mittel ein Polyamin-Derivat der allgemeinen Formel II ist:
R-X-L-Polyamin (II)
worin R eine geradkettige oder verzweigte, gesättigte oder ungesättigte aliphatische C₁₀₋₅₀-Gruppe, -Carboxyalkyl-, -Hydrocarboxyalkyl- oder -Alkoxy-Gruppe, eine alicyclische C₃₋₈-Gruppe, eine monocyclische oder polycyclische Arylsubstituierte oder unsubstituierte aliphatische Gruppe; eine aliphatisch substituierte oder unsubstituierte monocyclische oder polycyclische aromatische Gruppe; eine monocyclische oder polycyclische heterocyclische Gruppe; eine monocyclische oder polycyclische heterocyclische substituierte aliphatische Gruppe; ein Arylsulfonyl ist;
X -CO-, -SO₂- oder -CH₂- ist;
L eine kovalente Bindung oder eine natürlich vorkommende Aminosäure, Ornithin, 2,4-Diaminobuttersäure oder Derivate davon ist und
"Polyamin" ein natürlich vorkommendes oder synthetisch produziertes Polyamin ist.

2. Kosmetisches Verfahren gemäß Anspruch 1, das außerdem Rasieren der Haut umfasst.

3. Kosmetisches Verfahren gemäß einem der vorangehenden Ansprüche, wobei das Mittel DFMO ist.

4. Kosmetisches Verfahren gemäß einem der vorangehenden Ansprüche, wobei die Zusammensetzung außerdem einen Talgproduktions-Inhibitor umfasst, der ausgewählt ist aus der Gruppe, bestehend aus Carboxylaten von verzweigten Alkoholen und/oder Ethoxylaten davon, Triclosan, Cerulenin, Epigallocatechingallat (EGCG), α-Methylen-γ-butyrolacton, Gemischen davon und Analoga davon.

5. Kosmetisches Verfahren gemäß einem der vorangehenden Ansprüche, wobei das Verfahren eine Synthese von Squalen, Triglyceriden, freien Fettsäuren, Wachsestern, Cholesterin, Sterolestern und/oder einer Kombination davon reduziert.

6. Kosmetische Zusammensetzung zur Verminderung der Talgproduktion und/oder der Porengröße, umfassend:
eine die Talgproduktion und/oder Porengröße inhibierende wirksame Menge eines Mittels oder mehrerer Mittel, ausgewählt aus der Gruppe, bestehend aus Polyamin-Derivaten; DFMO; einem kosmetisch verträglichen Salz davon; einem Solvat davon und Gemischen davon, und einen kosmetisch verträglichen Träger, und
einen Talgproduktions-Inhibitor, ausgewählt aus der Gruppe, bestehend aus Carboxylaten von verzweigten Alkoholen und/oder Ethoxylaten davon, Triclosan, Cerulinin, Epigallocatechingallat (EGCG), α-Methylen-γ-butyrolacton, Gemischen davon,
wobei das Mittel ein Polyamin-Derivat der allgemeinen Formel I ist:
R-X-Polyamin (I)
worin R ausgewählt ist aus H und der Gruppe aus einer geradkettigen oder verzweigten, gesättigten oder ungesättigten, aliphatischen C₁₋₅₀-Gruppe, -Carboxyalkyl-, -Hydroxycarboxyalkyl- oder -Alkoxy-Gruppe; einer alicyclischen C₃₋₈-Gruppe, einer monocyclischen oder polycyclischen Aryl-substituierten aliphatischen Gruppe; einer aliphatisch substituierten monocyclischen oder polycyclischen aromatischen Gruppe; einer monocyclischen oder polycyclischen heterocyclischen Gruppe; einer monocyclischen oder polycyclischen heterocyclischen substituierten aliphatischen Gruppe; einem C₁₋₁₀-Alkyl; einem Arylsulfonyl und Cyano;
"X" -CO-, -SO₂- oder -CH₂- ist, und
"Polyamin" ein natürlich vorkommendes oder synthetisch produziertes Polyamin ist oder
wobei das Mittel ein Polyamin-Derivat der allgemeinen Formel II ist:
R-X-L-Polyamin (II)
worin R eine geradkettige oder verzweigte, gesättigte oder ungesättigte aliphatische C₁₀₋₅₀-Gruppe, -Carboxyalkyl-, -Hydrocarboxyalkyl- oder -Alkoxy-Gruppe; eine alicyclische C₃₋₈-Gruppe, eine monocyclische oder polycyclische Aryl-substituierte oder unsubstituierte aliphatische Gruppe; eine aliphatisch substituierte oder unsubstituierte monocyclische oder polycyclische aromatische Gruppe; eine monocyclische oder polycyclische heterocyclische Gruppe; eine monocyclische oder polycyclische heterocyclische substituierte aliphatische Gruppe; ein Arylsulfonyl ist;
X -CO-, -SO₂- oder -CH₂- ist;
L eine kovalente Bindung oder eine natürlich vorkommende Aminosäure, Ornithin, 2,4-Diaminobuttersäure oder ein Derivat davon ist und
"Polyamin" ein natürlich vorkommendes oder synthetisch produziertes Polyamin ist.

7. Kosmetische Zusammensetzung gemäß Anspruch 6, wobei das Mittel Difluorylmethylornithin (DFMO) ist.

8. Kosmetische Zusammensetzung gemäß Anspruch 6 oder Anspruch 7, wobei der Haarwachstums-Inhibitor 0,0001 Gewichts-% bis 50 Gewichts-% der Zusammensetzung ausmacht.

9. Kosmetische Zusammensetzung gemäß einem der Ansprüche 6 bis 8, das außerdem ein Mittel mit günstigen Eigenschaften für die Haut umfasst.

10. Kosmetisches System zur Verminderung der Talgproduktion in Haut, umfassend die kosmetische Zusammensetzung gemäß einem der Ansprüche 6 bis 9, einen Behälter für die Zusammensetzung und Instruktionen zur Anwendung der Zusammensetzung auf die Haut.

## Revendications

1. Procédé cosmétique permettant de diminuer la production de sébum dans les sébocytes et/ou la taille des pores de la peau d'un individu en ayant besoin, comprenant la mise en contact dudit sébocyte et/ou de ladite peau avec une composition comprenant une quantité inhibant la production de sébum et/ou la taille des pores d'un agent choisi dans le groupe constitué par les dérivés de polyamine ; la difluorylméthylornithine (DFMO) ; leurs sels acceptables en cosmétique ; leurs solvates ; et des mélanges de ceux-ci,
dans lequel ledit dérivé de polyamine est un composé de formule générale I :
R-X-polyamine (I)
dans laquelle R est choisi parmi H ou un groupe aliphatique, carboxyalkyle, hydroxycarboxyalkyle ou alcoxy, saturé ou insaturé en C₁₋₅₀, linéaire ou ramifié ; un groupe alicyclique en C₃₋₈ ; un groupe aliphatique portant un substituant aryle à un seul noyau ou à plusieurs noyaux ; un groupe aromatique à un seul noyau ou à plusieurs noyaux portant un substituant aliphatique ; un groupe hétérocyclique à un seul noyau ou à plusieurs noyaux ; un groupe aliphatique portant un substituant hétérocyclique à un seul noyau ou à plusieurs noyaux ; un groupe alkyle en C₁₋₁₀ ;un groupe arylsulfonyle ;ou un groupe cyano ;
"X" est -CO-, -SO₂- ou -CH₂- ; et
"polyamine" désigne une polyamine existant dans la nature ou produite par synthèse, ou
dans laquelle ledit agent est un dérivé de polyamine de formule générale II :
R-X-L-polyamine (II)
dans laquelle R est un groupe aliphatique, carboxyalkyle, hydroxycarboxyalkyle ou alcoxy, saturé ou insaturé en C₁₀₋₅₀, linéaire ou ramifié ; un groupe alicyclique en C₃₋₈ ; un groupe aliphatique portant ou non un substituant aryle à un seul noyau ou à plusieurs noyaux ; un groupe aromatique à un seul noyau ou à plusieurs noyaux, portant ou non un substituant aliphatique ; un groupe hétérocyclique à un seul noyau ou à plusieurs noyaux ; un groupe aliphatique portant un substituant hétérocyclique à un seul noyau ou à plusieurs noyaux ; un groupe arylsulfonyle ;
X est -CO-, -SO₂- ou -CH₂- ;
L est une liaison covalente ou un acide aminé existant dans la nature, l'ornithine, l'acide 2,4-diaminobutyrique, ou un de leurs dérivés ; et
"polyamine" désigne une polyamine existant dans la nature ou produite par synthèse.

2. Procédé cosmétique selon la revendication 1, comprenant en outre le rasage de ladite peau.

3. Procédé cosmétique selon l'une quelconque des revendications précédentes, dans lequel ledit agent est la DFMO.

4. Procédé cosmétique selon l'une quelconque des revendications précédentes, dans lequel la composition comprend en outre un inhibiteur de la production de sébum choisi dans le groupe constitué par les carboxyalkylates d'alcools ramifiés et/ou leurs éthoxylates, le triclosan, la cérulénine, le gallate d'épigallocatéchine (EGCG), l'α-méthylène-γ-butyrolactone, des mélanges de ceux-ci, et leurs analogues.

5. Procédé cosmétique selon l'une quelconque des revendications précédentes, dans lequel ledit procédé réduit la synthèse du squalène, des triglycérides, des acides gras libres, des esters de cire, du cholestérol, des esters de stérol, et/ou d'une combinaison de ceux-ci.

6. Composition cosmétique permettant de réduire la production de sébum et/ou la taille des pores comprenant :
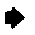 une quantité, efficace pour inhiber la production de sébum et/ou la taille des pores, d'un ou plusieurs agents choisis dans le groupe constitué par les dérivés de polyamine ; la DFMO ; un de leurs sels acceptables en cosmétique ; un de leurs solvates ; et des mélanges de ceux-ci ; et
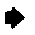 un véhicule acceptable en cosmétique, et
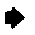 un inhibiteur de la production de sébum choisi dans le groupe constitué par les carboxyalkylates d'alcools ramifiés et/ou leurs éthoxylates, le triclosan, la cérulinine, le gallate d'épigallocatéchine (EGCG), l'α-méthylène-γ-butyrolactone, des mélanges de ceux-ci,
dans laquelle ledit agent est un dérivé de polyamine de formule générale I :
R-X-polyamine (I)
dans laquelle R est choisi parmi H ou un groupe aliphatique, carboxyalkyle, hydrocarboxyalkyle ou alcoxy, saturé ou insaturé en C₁₋₅₀, linéaire ou ramifié ; un groupe alicyclique en C₃₋₈ ; un groupe aliphatique portant un substituant aryle à un seul noyau ou à plusieurs noyaux ; un groupe aromatique à un seul noyau ou à plusieurs noyaux portant un substituant aliphatique ; un groupe hétérocyclique à un seul noyau ou à plusieurs noyaux ; un groupe aliphatique portant un substituant hétérocyclique à un seul noyau ou à plusieurs noyaux ; un groupe alkyle en C₁₋₁₀ ;un groupe arylsulfonyle ;ou un groupe cyano ;
"X" est -CO-, -SO₂- ou -CH₂- ; et
"polyamine" désigne une polyamine existant dans la nature ou produite par synthèse, ou
dans laquelle ledit agent est un dérivé de polyamine de formule générale II :
R-X-L-polyamine (II)
dans laquelle R est un groupe aliphatique, carboxyalkyle, hydroxycarboxyalkyle ou alcoxy, saturé ou insaturé en C₁₀₋₅₀, linéaire ou ramifié ; un groupe alicyclique en C₃₋₈ ; un groupe aliphatique portant ou non un substituant aryle à un seul noyau ou à plusieurs noyaux ; un groupe aromatique à un seul noyau ou à plusieurs noyaux, portant ou non un substituant aliphatique ; un groupe hétérocyclique à un seul noyau ou à plusieurs noyaux ; un groupe aliphatique portant un substituant hétérocyclique à un seul noyau ou à plusieurs noyaux ; un groupe arylsulfonyle ;
X est -CO-, -SO₂- ou -CH₂- ;
L est une liaison covalente ou un acide aminé existant dans la nature, l'ornithine, l'acide 2,4-diaminobutyrique, ou un de leurs dérivés ; et
"polyamine" désigne une polyamine existant dans la nature ou produite par synthèse.

7. Composition cosmétique selon la revendication 6, dans laquelle ledit agent est la difluorylméthylornithine (DFMO).

8. Composition cosmétique selon la revendication 6 ou la revendication 7 dans laquelle ledit inhibiteur de la pousse des cheveux constitue de 0,0001% à 50% en poids de ladite composition.

9. Composition cosmétique selon l'une quelconque des revendications 6 à 8, comprenant en outre un agent bon pour la peau.

10. Système cosmétique permettant de réduire la production de sébum dans la peau, comprenant ladite composition cosmétique selon l'une quelconque des revendications 6 à 9, un récipient pour ladite composition, et un mode d'emploi pour appliquer ladite composition sur ladite peau.
